# EUROPÄISCHE PATENTSCHRIFT

(11) **EP 0 434 619 B1**
(45) Veröffentlichungstag und Bekanntmachung des Hinweises auf die Patenterteilung: **06.09.1995**
(21) Anmeldenummer: 90810978.8
(22) Anmeldetag: 12.12.1990
(51) Int. Cl.: C08F 20/36, C08G 85/00, C08L 101/00, C09D 139/04, C08F 2/44, C09D 157/04

(54) **Verfahren zum Einbau von O-Hydroxyphenyl-S-triazinen in organische Polymere**
Process for incorporating O-hydroxyphenyl-S-triazin into organic polymers
Procédé pour incorporer des groupes O-hydroxyphényl-S-triazine dans des polymères organiques

(30) Priorität: 21.12.1989 CH 4581/89
(43) Veröffentlichungstag der Anmeldung: 26.06.1991
(73) Patentinhaber: Ciba Specialty Chemicals Holding Inc., 4057 Basel (CH)
(72) Erfinder: Birbaum, Jean-Luc, Dr., CH-1700 Fribourg (CH); Rody, Jean, Dr., CH-4125 Riehen (CH); Slongo, Mario, Dr., CH-1712 Tafers (CH); Valet, Andreas, Dr., W-7859 Eimeldingen (DE)

(56) Entgegenhaltungen:
- EP-A- 0 000 496
- CH-A- 481 954
- US-A- 3 423 360
- US-A- 4 356 287

## Beschreibung

Die vorliegende Erfindung betrifft ein Verfahren zum Einbau von o-Hydroxyphenyl-s-triazinen in organische Polymere und die nach diesem Verfahren erhältlichen modifizierten Polymeren.

Es ist bekannt, dass o-Hydroxyphenyl-s-triazine UV-Licht absorbieren und auf Grund dieser Eigenschaft als Lichtschutzmittel für organische Materialien, insbesondere für organische Polymere verwendet werden können. Insbesondere werden dazu solche Verbindungen verwendet, die ausser der o-Hydroxygruppe noch eine p-Alkoxygruppe enthalten, wobei die Alkoxygruppe auch substituiert sein kann, wie dies z.B. im US-A-3 118 887 oder im US-A-3 244 708 beschrieben ist.

Um ein Auswandern und Ausblühen dieser Stabilisatoren zu verhindern, hat man auch bereits vorgeschlagen, p-Acryloyloxyderivate von o-Hydroxyphenyl-s-triazinen in die zu schützenden Polymeren durch Copolymerisation oder durch Pfropfung einzubauen, wie dies im US-A-3 423 360 beschrieben ist. Diese p-Acryloyloxyderivate neigen jedoch zu einer Vergilbung unter dem Einfluss von UV-Licht.

Auf der Suche nach chemisch einbaubaren o-Hydroxyphenyl-s-triazin-Derivaten wurde eine grössere Anzahl von Derivaten gefunden, die sich durch Copolymerisation, Copolykondensation, Copolyaddition oder polymeranaloge Umsetzung in verschiedene Typen von organischen Polymeren einbauen lassen und die im eingebauten Zustand weniger Zu Verfärbungen neigen als die vorhin erwähnten bekannten Derivate.

Die Erfindung betrifft daher ein Verfahren zum Einbau von o-Hydroxyphenyl-s-triazinen in organische Polymere, dadurch gekennzeichnet, dass man eine Verbindung der Formel I, 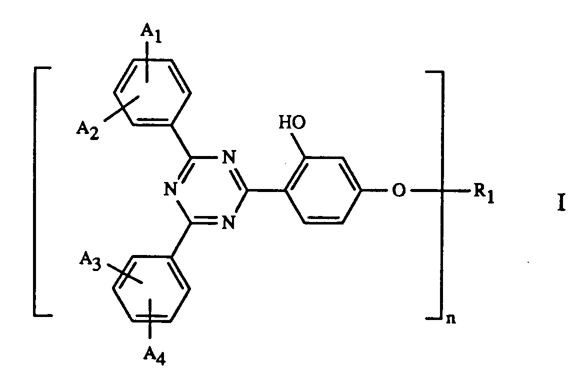
worin n 1 oder 2 ist,
A₁, A₂, A₃ und A₄ unabhängig voneinander Wasserstoff, C₁-C₁₂-Alkyl, Cyclohexyl oder Halogen bedeuten,
R₁, wenn n 1 ist, Wasserstoff, durch OH, -COOH, -COOR₂, -NHR₃, -CONHR₄, 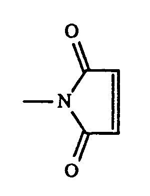
oder/und -O-CO-R₄ substituiertes C₁-C₁₈-Alkyl, durch OH substituiertes und durch ein oder mehrere O unterbrochenes C₄-C₂₀-Alkyl, durch OH und C₁-C₁₂-Alkoxy oder Phenoxy substituertes C₂-C₄-Alkyl, durch OH oder -OCOR₄ substituiertes Cyclohexyl, C₂-C₆-Alkenyl, Glycidyl oder eine der Gruppen 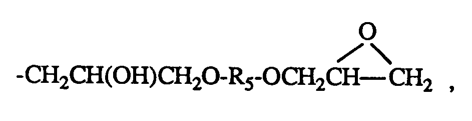
-CO-R₆-COOH oder -CO-NH-R₇-NCO bedeutet,
und wenn n 2 ist, eine der Gruppen -CH₂CH(OH)CH₂-, -CO-CH=CH-CO-,
-CH₂CH(OH)CH₂O-R₅-OCH₂CH(OH)CH₂- oder
-CH₂CH(R₈)O-CO-CH=CH-CO-OCH(R₈)CH₂- bedeutet,
R₂ C₁-C₄-Alkyl, Glycidyl oder C₃-C₅-Alkenyl bedeutet,
R₃ Wasserstoff, C₁-C₁₂-Alkyl, C₃-C₅-Alkenyl oder Cyclohexyl bedeutet,
R₄ C₂-C₆-Alkenyl oder C₂-C₆-Hydroxyalkyl bedeutet,
R₅ C₂-C₁₀-Alkylen, Phenylen oder eine Gruppe 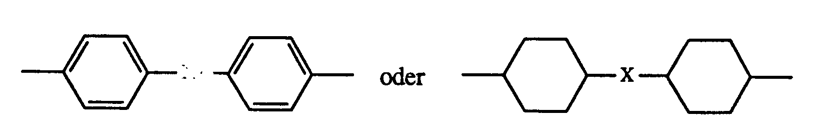
bedeutet, worin X -O-, -S-, -SO₂, -CH₂- oder -C(CH₃)₂- ist, oder R₅ eine Gruppe -CO-R₉-CO- bedeutet,
R₆ C₂-C₁₄-Alkylen, -CH=CH- oder o-Phenylen bedeutet,
R₇ C₂-C₁₀-Alkylen, Phenylen, Tolylen oder eine Gruppe der Formel 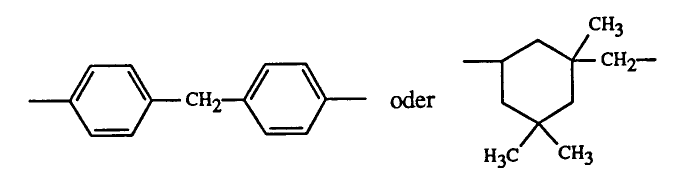
bedeutet,
R₈ Wasserstoff oder Methyl bedeutet und
R₉ C₂-C₁₀-Alkylen, -CH=CH- oder Phenylen bedeutet,
entweder bei der Herstellung des Polymeren durch Copolymerisation, Copolykondensation oder Copolyaddition einbaut oder mit einem Polymeren, das geeignete funktionelle Gruppen enthält, reagieren lässt.

A₁, A₂, A₃, A₄ und R₃ als C₁-C₁₂-Alkyl können unverzweigtes oder verzweigtes Alkyl sein, wie z.B. Methyl, Ethyl, iso-Propyl, n-Butyl, sec-Butyl, tert-Butyl, Pentyl, Hexyl, Heptyl, Octyl, 2-Ethylhexyl, ditert-Octyl, Decyl oder Dodecyl.

R₁ und R₄ als C₂-C₆-Alkenyl kann z.B. Vinyl, 1-Propenyl, Allyl, Methallyl, 2-Butenyl oder 2-Hexenyl sein. R₂ und R₃ als C₃-C₅-Alkenyl sind insbesondere Allyl oder Methallyl. R₂ als C₁-C₄-Alkyl ist insbesondere Methyl oder Ethyl.

R₄ als C₂-C₆-Hydroxyalkyl ist insbesondere 2-Hydroxyethyl oder 2-Hydroxypropyl.

R₅, R₇ und R₉ als C₂-C₁₀-Alkylen kann z.B. 1,2-Ethylen, Trimethylen, Tetramethylen, Hexamethylen, Octamethylen oder 1,3,3-Trimethyltetramethylen sein. R₆ als C₂-C₁₄ Alkylen ist vorzugsweise 1,2-Alkylen wie z.B. 1,2-Ethylen, 1,2-Propylen, 1,2-Octylen oder 1,2-Dodecylen.
R₁ als substituiertes C₁-C₁₈-Alkyl ist vorzugsweise substituiertes C₁-C₄-Alkyl. Es kann einen oder mehrere der Substituenten OH, -COOH, -COOR₂, -NHR₃, -CONHR₄ und -O-CO-R₄ enthalten. Beispiele hierfür sind 2-Hydroxyethyl, 2-Hydroxypropyl, 2-Hydroxybutyl, Carboxymethyl, 2-Carboxyethyl, 2-Carboxypropyl, Methoxycarbonylmethyl, Methoxycarbonylethyl, Ethoxycarbonylethyl, Glycidyloxycarbonylethyl, Allyloxycarbonylmethyl, Allyloxycarbonylethyl, 2-Aminoethyl, 2-Hydroxy-3-methylaminopropyl, 2-Hydroxy-3-butylaminopropyl, 2-Hydroxy-3-allylaminopropyl, 2-(Allylaminocarbonyl)-ethyl, 2-Hydroxyethylaminocarbonylmethyl, 2-Acryloyloxyethyl, Methacryloyloxymethyl, 2-Hydroxy-3-acryloyloxypropyl, 2-Hydroxy-3-methacryloyloxypropyl, 2-Hydroxy-3-maleinimido-propyl oder 2,3-Dihydroxypropyl.

Wenn R₁ ein durch OH substituiertes und durch ein oder mehrere O unterbrochenes C₄-C₂₀-Alkyl ist, so kann dies z.B. eine Gruppe 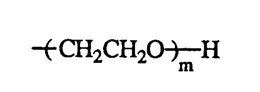
mit m = 2-10 oder eine Gruppe 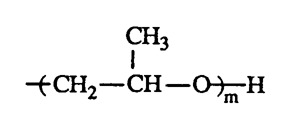
mit m = 2-6 sein.

Wenn R₁ ein durch OH und C₁-C₁₂-Alkoxy oder Phenoxy substituiertes C₂-C₄-Alkyl ist, so ist dies insbesondere solcherart substituiertes Propyl, wie z.B. 2-Hydroxy-2-butoxypropyl, 2-Hydroxy-3-hexyloxypropyl, 2-Hydroxy-3-octyloxypropyl, 2-Hydroxy-3-dodecyloxypropyl oder 2-Hydroxy-3-phenoxypropyl.

Wenn R₁ ein durch OH oder -OCOR₄ substituiertes Cyclohexyl ist, so steht die Gruppe OH oder -OCOR₄ bevorzugt in ortho-Stellung (2-Stellung).

Bevorzugt verwendet man zum Einbau eine Verbindung der Formel I, worin n 1 oder 2 ist, A₁, A₂, A₃ und A₄ Wasserstoff, C₁-C₄-Alkyl oder Chlor bedeuten,
R₁, wenn n 1 ist, Wasserstoff, durch OH, -COOH, -COOR₂, -NHR₃, -CONHR₄ oder/und -O-CO-R₄ substituiertes C₁-C₄-Alkyl, durch OH substituiertes und durch ein oder mehrere O unterbrochenes C₄-C₂₀-Alkyl, durch OH und C₁-C₁₂-Alkoxy oder Phenoxy substituertes Propyl, durch OH oder -OCOR₄ substituiertes Cyclohexyl, Allyl, Glycidyl oder eine der Gruppen 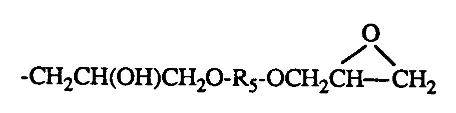
oder -CO-NH-R₇-NCO bedeutet, und wenn n 2 ist, eine der Gruppen -CH₂CH(OH)CH₂-,
-CH₂CH(OH)CH₂O-R₅-OCH₂CH(OH)CH₂- oder
-CH₂CH(R₈)O-CO-CH=CH-CO-OCH(R₈)CH₂- bedeutet,
R₂ C₁-C₄-Alkyl, Glycidyl oder Allyl bedeutet,
R₃ C₁-C₁₂-Alkyl bedeutet,
R₄ C₂-C₆-Alkenyl bedeutet,
R₅ C₂-C₁₀-Alkylen, Phenylen oder eine Gruppe 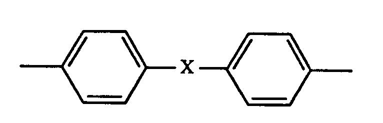
bedeutet, worin X -O-, -S-, -SO₂, -CH₂- oder -C(CH₃)₂- ist, oder R₅ eine Gruppe -CO-R₉-CO- bedeutet,
R₇ C₂-C₁₀-Alkylen, Phenylen, Tolylen oder eine Gruppe der Formel 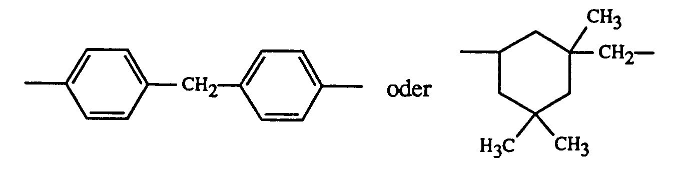
bedeutet,
R₈ Wasserstoff oder Methyl bedeutet und
R₉ C₂-C₁₀-Alkylen, -CH=CH- oder Phenylen bedeutet.

Besonders bevorzugt verwendet man zum Einbau eine Verbindung der Formel I, worin n 1 oder 2 ist,
A₁ und A₃ Wasserstoff, Methyl oder Chlor bedeuten,
A₂ und A₄ Wasserstoff oder Methyl bedeuten,
R₁, wenn n 1 ist, Wasserstoff, durch OH, -COOH, -COOR₂ oder/und -O-CO-R₄ substituiertes C₁-C₄-Alkyl, durch OH substituiertes und durch ein oder mehrere O unterbrochenes C₄-C₂₀-Alkyl, durch OH und C₄-C₁₂-Alkoxy substituiertes Propyl, Glycidyl, Allyl oder eine Gruppe 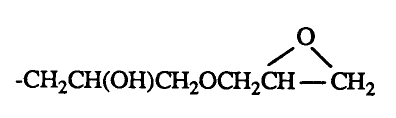
bedeutet, und wenn n 2 ist, eine der Gruppen -CH₂CH(OH)CH₂- oder
-CH₂CH(OH)CH₂O-R₅-OCH₂CH(OH)CH₂- bedeutet,
R₂ C₁-C₄-Alkyl oder Allyl bedeutet,
R₄ C₂-C₄-Alkenyl bedeutet und
R₅ C₄-C₈-Alkylen oder 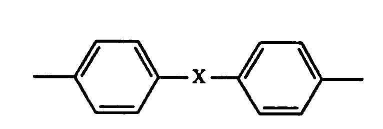
bedeutet, worin X -CH₂- oder -C(CH₃)₂- ist.

Der Einbau kann durch Copolymerisation, Copolykondensation oder Copolyaddition erfolgen oder durch Reaktion mit einem Polymeren, das geeignete funktionelle Gruppen trägt.

Der Einbau durch Copolymerisation kommt vor allem für solche Verbindungen der Formel I in Frage, die ethylenisch ungesättigte Gruppen enthalten. Dies sind die Verbindungen der Formel I, worin n 1 oder 2 ist, R₁, wenn n 1 ist, C₂-C₆-Alkenyl, durch -COOR₂, -NH-R₃, -CONHR₄ oder -O-CO-R₄ substituiertes C₁-C₁₈-Alkyl, durch -O-CO-R₄ substituiertes Cyclohexyl oder eine Gruppe -CO-CH=CH-COOH bedeutet, und wenn n = 2 ist, eine Gruppe -CO-CH=CH-CO-,
-CH₂CH(R₈)O-CO-CH=CH-CO-OCH(R₈)CH₂- oder
-CH₂CH(OH)CH₂O-CO-CH=CH-CO-OCH₂CH(CH)CH₂- bedeutet, R₂ C₃-C₅-Alkenyl ist, R₃ Allyl ist, R₄ C₂-C₆-Alkenyl ist und R₈ Wasserstoff oder Methyl ist.

Besonders geeignet sind hierzu Verbindungen der Formel I, worin R₁ Allyl, durch -COOR₂ oder -O-CO-R₄ substituiertes C₁-C₄-Alkyl oder durch -O-CO-R₄ substituiertes Cyclohexyl bedeutet, R₂ Allyl ist und R₄ C₂-C₄-Alkenyl bedeutet.

Der Einbau durch Copolymerisation kann in solche Polymere erfolgen, die durch Polymerisation ethylenisch ungesättigter Monomere hergestellt werden. Dazu zählen beispielsweise folgende Monomere:
Acrylsäure, Methacrylsäure, Ester der Acryl- und Methacrylsäure, Amide der Acryl- und Methacrylsäure, Acrylnitril, Styrol, α-Methylstyrol, Butadien, Isopren, Maleinsäureanhydrid, Ester, Amide und Imide der Maleinsäure, Vinylchlorid, Vinylidenchlorid, Vinylacetat, Vinylbutyral, Vinylalkylether oder N-Vinylpyrrolidon. Bevorzugt ist der Einbau in solche Polymere, die aus Acrylsäure, Methacrylsäure, Estern oder Amiden der Acryl- oder Methacrylsäure, Styrol oder Acrylnitril aufgebaut sind. Das Polymer kann aus einem oder mehreren solcher Monomeren aufgebaut sein. Die Zugabe der ungesättigten Verbindung der Formel I erfolgt während der Polymerisation, sodass eine Copolymerisation stattfindet.

Die Polymerisation kann durch radikalische, anionische oder kationische Initiatoren initiert werden. Vorzugsweise verwendet man radikalische Initiatoren, die beim Erwärmen in Radikale zerfallen, wie z.B. organische Peroxide oder Hydroperoxide, Azoverbindungen oder Redox-Katalysatoren. Die Polymerisation kann auch durch energiereiche Strahlung initiert werden, so z.B. auch bei strahlenhärtbaren Lacken (UV-härtbar oder ESR-härtbar). Dabei wird das copolymerisierbare Hydroxyphenyl-s-triazin während der Filmbildung in die Lackmatrix eingebaut.

Besonders geeignet für solche Copolymerisationen ist das Verfahren der Gruppentransferpolymerisation. Hierbei wird durch Verwendung bestimmter Initiatoren ein "lebendes" Polymer gebildet. Beispiele für hierfür geeignete Initiatoren sind 1-Trimethylsiloxy-1-alkoxy-2-methylpropene. Das Verfahren der Gruppentransferpolymerisation ist seit einigen Jahren bekannt und beispielsweise in US-A-4 695 607 und US-A-4 414 372 beschrieben.

Die Polymerisation kann in Lösung, Emulsion, Dispersion oder in Masse ausgeführt werden. Dem Fachmann sind diese Verfahren bekannt. In den später folgenden Beispielen werden solche Copolymerisationen im Detail beschrieben.

Für den Einbau durch Copolykondensation oder Copolyaddition kommen vor allem solche Verbindungen der Formel I in Frage, die zwei funktionelle Gruppen enthalten. Dies sind z.B. die Verbindungen der Formel I, worin n 1 oder 2 ist, R₁, wenn n 1 ist, durch OH und -COOH, -COOR₂ oder -NHR₃ substituiertes C₁-C₁₈-Alkyl oder eine Gruppe -CH₂CH(OH)CH₂OH oder 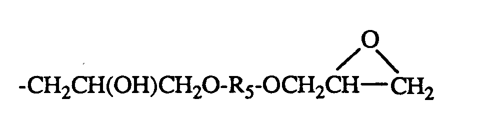
bedeutet, und wenn n 2 ist, eine Gruppe -CH₂CH(OH)CH₂O-R₅-OCH₂CH(OH)CH₂- bedeutet, R₂ C₁-C₄-Alkyl bedeutet, R₃ Wasserstoff, C₁-C₁₂-Alkyl, Allyl oder Cyclohexyl bedeutet und R₅ C₂-C₁₀-Alkylen, Phenylen oder eine Gruppe 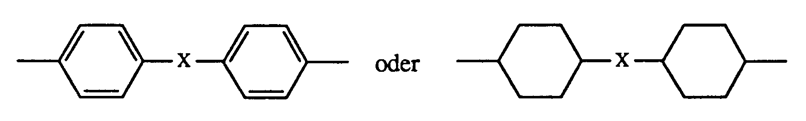
bedeutet,
worin X -O-, -S-, -SO₂-, -CH₂- oder -CH(CH₃)₂- ist. Sollen nur kleine Mengen einer Verbindung der Formel I durch Polykondensation oder Polyaddition eingebaut werden, so kommen auch solche Verbindungen der Formel I in Frage, die nur eine funktionelle Gruppe wie z.B. eine Gruppe -OH, -COOH, -COOR₂, -NHR₃ oder 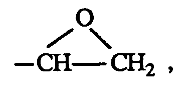
enthalten.

Dies sind insbesondere solche Verbindungen der Formel I, worin n 1 ist,
R₁ Wasserstoff, durch OH, -COOH, -COOR₂, -NHR₃, -CONHR₄ oder -O-CO-R₄ substituiertes C₁-C₁₈-Alkyl, durch OH substituiertes und durch ein oder mehrere O unterbrochenes C₄-C₂₀-Alkyl, durch OH und C₁-C₁₂-Alkoxy oder Phenoxy substituiertes C₂-C₄-Alkyl, durch OH substituiertes Cyclohexyl, Glycidyl oder eine der Gruppen -CO-R₆-COOH oder -CO-NH-R₇-NCO bedeutet,
R₂ C₁-C₄-Alkyl oder Glycidyl bedeutet, R₃ Wasserstoff, C₁-C₁₂-Alkyl oder Cyclohexyl bedeutet, R₄ C₂-C₆-Hydroxyalkyl bedeutet, R₆ C₂-C₁₄-Alkylen, -CH=CH- oder o-Phenylen bedeutet und R₇ die in Anspruch 1 gegebenen Bedeutungen hat.

Solche mono- oder difunktionelle Verbindungen der Formel I können z.B. in Polyester, Polyetherester, Polyamide, Polyurethane, Polycarbonate, Epoxidharze, Phenolharze, Melaminharze oder Alkydharze eingebaut werden. Der Einbau erfolgt durch Zugabe bei der Herstellung der Kondensations- oder Additionspolymeren nach den hierfür dem Fachmann bekannten Methoden.

Der Einbau kann auch in oligomere oder polymere Zwischenprodukte stattfinden. Z.B. können ungesättigte Verbindungen der Formel I zu ungesättigten Polyesterharzen und deren Gemischen mit anderen Vinylverbindungen zugesetzt werden und dann unter Zugabe radikalischer Initiatoren ausgehärtet werden. Oder oligomere Epoxidharze können mit funktionellen Verbindungen der Forme I umgesetzt werden und anschliessend mit einem Epoxid-Härter ausgehärtet werden. Oder OH-funktionelle Verbindungen der Formel I können mit Melaminharzen umgesetzt werden und die dabei entstandenen Verbindungen anschliessend unter Zugabe von Acrylatharzen ausgehärtet werden.

Auch der Einbau in Polyurethane, Phenolharze oder Alkydharze kann mit einer Vorstufe (precursor) geschehen, vor der endgültigen Aushärtung des Harzes. Die Aushärtung des Harzes kann auch unter saurer oder basischer Katalyse geschehen ohne dass dabei die Triazinverbindungen stören.

Eine weitere Möglichkeit des Einbaus besteht in der Reaktion einer Verbindung der Formel I mit einem Polymeren, das geeignete funktionelle Gruppen enthält. Dies können z.B. Polymere mit Hydroxyl-, Carboxyl-, Anhydrid-, Amino-, Epoxid- oder Isocyanatgruppen sein. Beispiele hierfür sind Copolymerisate von Acryl- und Methacrylsäure, von Hydroxyalkyl(meth)acrylaten, von Glycidyl(meth)acrylaten, partiell hydrolysiertes Polyvinylacetat oder Ethylen-vinylacetat-Copolymer partiell veresterte Cellulose, partiell hydrolysierte Polyalkyl(meth)acrylate, Polyester oder Polyurethane mit reaktiven Endgruppen, Epoxidharze oder Copolymere der Maleinsäure, des Maleinsäureanhydrides oder von Maleinsäurehalbestern oder -halbamiden.

Für die Umsetzung kommen solche Verbindungen der Formel I in Frage, die eine funktionelle Gruppe enthalten, die mit den funktionellen Gruppen des Polymeren reagieren kann. Dies können z.B. Hydroxyl- Carboxyl-, Ester-, Amino-, Epoxid- oder Isocyanatgruppen sein. Wenn man z.B. ein Polymeres, das OH-Gruppen enthält, mit einer Verbindung der Formel I modifizieren will, so kann man beispielsweise eine Verbindung der Formel I verwenden, die mindestens eine Isocyanat-, Epoxid-, Carboxyl- oder Estergruppe enthält. Ein Polymer, das Epoxidgruppen enthält, kann z.B. mit einer Verbindung der Formel I umgesetzt werden, die mindestens eine Hydroxyl-, Carboxyl- oder Aminogruppe enthält. Ein Copolymer des Maleinsäureanhydrides kann z.B. mit einer Verbindung der Formel I umgesetzt werden, die eine Hydroxyl-, Amine oder Epoxidgruppe enthält.

Diese Reaktionen werden nach den allgemein üblichen Methoden für polymerhomologe Umsetzungen ausgeführt. Vorzugsweise wird die Reaktion in Lösung ausgeführt. Man kann dabei alle funktionellen Gruppen des Polymeren oder nur einen Teil davon umsetzen. Danach richtet sich die Menge der zur Umsetzung verwendeten Verbindung der Formel I. In den später folgenden Beispielen werden Details für solche Umsetzungen beschrieben.

Eine spezielle Methode zum Einbau in Polymere ist die Pfropfung von ethylenisch ungesättigten Derivaten der Formel I auf Kohlenwasserstoff-Polymeren. Die Verbindungen der Formel I, die ethylenisch ungesättigte Gruppen enthalten, wurden vorhin bereits näher definiert. Kohlenwasserstoff-Polymere können gesättigt oder ungesättigt sein. Zu ersteren gehören die Polyolefine, wie z.B. Polyethylen, Polypropylen, Polybuten oder Polyisobuten. Zu letzteren gehören die Dien-Polymeren und deren Copolymerisate mit Olefinen, wie z.B. Polybutadien, Polyisopren, Propylen-Butadien oder Ethylen-Propylen-Butadien. Bevorzugt ist die Pfropfung auf Polyolefine, insbesondere auf Polyethylen.

Die Pfropfreaktion kann in Lösung oder in Masse durchgeführt werden. Als Katalysatoren verwendet man Radikalbildner, wie sie auch für die Homo- oder Copolymerisation ungesättigter Verbindung verwendet werden.

Alle diese Verfahren zum Einbau von Verbindungen der Formel I in Polymere können mit einer relativ kleinen Menge des Triazines durchgeführt werden, beispielsweise mit 0,05 bis 5 Gew.-%, bezogen auf das modifizierte Polymere. Diese Mengen verleihen den Polymeren Beständigkeit gegen Schädigung durch Licht, Sauerstoff und Wärme, wobei diese Stabilisierung nicht durch Auswandern oder Eluieren des Stabilisators verlorengehen kann. Bevorzugt werden für diesen Zweck 0,1 bis 3 Gew.-% einer Verbindung der Formel I eingebaut.

Man kann nach diesen Verfahren aber auch grössere Mengen des Triazins einbauen, beispielsweise 5 bis 50 Gew.-% bezogen auf das modifizierte Polymere. Dies ist dann sinnvoll, wenn man die so modifizierten Polymeren als polymere Stabilisatoren verwenden will. Diese polymeren Stabilisatoren können organischen Materialien, insbesondere organischen Polymeren, zugesetzt werden. Die polymeren Stabilisatoren können aber auch als dünne Schutzschicht auf Kunststoff-Formteile aufgebracht werden, beispielsweise in gelöster Form oder durch Coextrusion wie es z.B. im US-A-4 676 870 beschrieben ist.

Eine weitere Anwendung dieser Verfahren ist der Einbau von Verbindungen der Formel I in Polymermikropartikel. In EP-A-226 538 ist der Einbau von Lichtschutzmitteln durch Copolymerisation oder Copolykondensation in solche Mikropartikel beschrieben, die als disperse Phase in Ueberzügen verwendet werden können. In diesem Falle baut man in die Mikropartikel 0,1 bis 30 Gew.-% einer Verbindung der Formel I ein, bevorzugt 0,5 bis 10 Gew.-%, bezogen auf das modifizierte Polymere.

Für den Einbau in Mikropartikel kann vorteilhafterweise das Verfahren der Gruppentransferpolymerisation verwendet werden, wie dies z.B. in der EP-A-293 871 beschrieben ist.

Die Erfindung betrifft auch die durch das geschilderte Verfahren hergestellten modifizierten Polymeren, die eine Verbindung der Formel I in den genannten Gewichtsmengen in chemisch gebundener Form enthalten und dadurch gegen Schädigung durch Licht, Sauerstoff und Wärme stabilisiert sind.

Die so modifizierten Polymeren können mit verschiedenen Zusätzen versetzt werden, wie dies in der Technologie der Polymeren üblich ist. Diese Zusätze können Stabilisatoren sein oder Verarbeitungshilfsmittel oder Pigmente oder sonstige Zusätze. Beispiele hierfür sind die folgenden Zusätze.
1. Antioxidantien
   1.1. Alkylierte Monophenole, z.B. 2,6-Di-tert.butyl-4-methylphenol, 2-Tert.butyl-4,6-dimethylphenol, 2,6-Di-tert.butyl-4-ethylphenol, 2,6-Di-tert.butyl-4-n-butylphenol, 2,6-Di-tert.butyl-4-i-butylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tert.butyl-4-methoxymethylphenol, 2,6-Di-nonyl-4-methylphenol.
   1.2. Alkylierte Hydrochinone, z.B. 2,6-Di-tert.butyl-4-methoxyphenol, 2,5-Di-tert.butyl-hydrochinon, 2,5-Di-tert.amyl-hydrochinon, 2,6-Diphenyl-4-octadecyloxyphenol.
   1.3. Hydroxylierte Thiodiphenylether, z.B. 2,2'-Thio-bis-(6-tert.-butyl-4-methylphenol), 2,2'-Thio-bis-(4-octylphenol), 4,4'-Thio-bis-(6-tert.butyl-3-methylphenol), 4,4'-Thio-bis-(6-tert.butyl-2-methylphenol).
   1.4. Alkyliden-Bisphenole, z.B. 2,2'-Methylen-bis-(6-tert.butyl-4-methylphenol), 2,2'-Methylen-bis-(6-tert.butyl-4-ethylphenol), 2,2'-Methylen-bis-[4-methyl-6-(α-methylcyclohexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-tert.butylphenol), 2,2'-Ethyliden-bis-(4,6-di-tert.butylphenol), 2,2'-Ethyliden-bis-(6-tert.butyl-4-isobutylphenol), 2,2'-Methylen-bis-[6-(α-methylbenzyl)-4-nonylphenol], 2,2'-Methylen-bis-[6-(α,α-dimethylbenzyl)-4-nonylphenol], 4,4'-Methylen-bis-(2,6-di-tert.-butylphenol), 4,4'-Methylen-bis-(6-tert.butyl-2-methylphenol), 1,1-Bis-(5-tert.butyl-4-hydroxy-2-methylphenyl)-butan, 2,6-Bis-(3-tert.butyl-5-methyl-2-hydroxybenzyl)-4-methylphenol, 1,1,3-Tris-(5-tert.butyl-4-hydroxy-2-methylphenyl)-butan, 1,1-Bis-(5-tert.butyl-4-hydroxy-2-methylphenyl)-3-n-dodecylmercaptobutan, Ethylenglycol-bis-[3,3-bis-(3'-tert.-butyl-4'-hydroxyphenyl)-butyrat], Bis-(3-tert.butyl-4-hydroxy-5-methyl-phenyl)-dicylopentadien, Bis-[2-(3'-tert.butyl-2'-hydroxy-5'-methyl-benzyl)-6-tert.butyl-4-methyl-phenyl]-terephthalat.
   1.5 Benzylverbindungen, z.B. 1,3,5-Tris-(3,5-di-tert.butyl-4-hydroxy-benzyl)-2,4,6-tri-methylbenzol, Bis-(3,5-di-tert.butyl-4-hydroxybenzyl)-sulfid, 3,5-Di-tert.butyl-4-hydroxybenzyl-mercaptoessigsäure-isooctylester, Bis-(4-tert.butyl-3-hydroxy-2,6-dimethylbenzyl)dithiol-terephthalat, 1,3,5-Tris-(3,5-di-tert.butyl-4-hydroxybenzyl)-isocyanurat, 1,3,5-Tris-(4-tert.butyl-3-hydroxy-2,6-dimethylbenzyl)-isocyanurat, 3,5-Di-tert.butyl-4-hydroxybenzyl-phosphonsäure-dioctadecylester, Ca-Salz des 3,5-Di-tert.butyl-4-hydroxybenzyl-phosphonsäure-monoethylester, 1,3,5-Tris-(3,5-dicyclohexyl-4-hydroxybenzyl)isocyanurat.
   1.6. Acylaminophenole, z.B. 4-Hydroxy-laurinsäureanilid, 4-Hydroxystearinsäureanilid, 2,4-Bis-(octylmercapto)-6-(3,5-di-tert.butyl-4-hydroxyanilino )-s-triazin, N-(3,5-di-tert.butyl-4-hydroxyphenyl)-carbaminsäureoctylester.
   1.7. Ester der β-(3,5-Di-tert.butyl-4-hydroxyphenyl)-propionsaure mit ein- oder mehrwertigen Alkoholen, wie z.B. mit Methanol, Octa- decanol, 1,6-Hexandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid.
   1.8. Ester der β-(5-tert.Butyl-4-hydroxy-3-methylphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid.
   1.9. Ester der β-(3,5-Dicyclohexyl-4-hydroxyphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid.
   1.10. Amide der β-(3,5-Di-tert.butyl-4-hydroxyphenyl)-propionsäure, wie z.B. N,N'-Bis-(3,5-di-tert.butyl-4-hydroxyphenylpropionyl)-hexamethylendiamin, N,N'-Bis-(3,5-di-tert.butyl-4-hydroxyphenylpropionyl)-trimethylendiamin, N,N'-Bis-(3,5-di-tert.butyl-4-hydroxyphenylpropionyl)-hydrazin.
2. UV-Absorber und Lichtschutzmittel
   2.1. 2-(2'-Hydroxyphenyl)-benztriazole, wie z.B. das 5'-Methyl-, 3',5'-Di-tert.butyl-, 5'-tert.-Butyl-, 5'-(1,1,3,3-Tetramethylbutyl)-, 5-Chlor-3',5'-di-tert.butyl-, 5-Chlor-3'-tert.butyl-5'-methyl-, 3'-sec.Butyl-5'-tert.butyl, 4'-Octoxy-, 3',5'-Di-tert.amyl-, 3',5'-Bis-(α,α-dimethylbenzyl)-Derivat.
   2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.
   2.3. Ester von gegebenenfalls substituierten Benzoesäuren, wie z.B. 4-tert.Butyl-phenylsalicylat, Phenylsalicylat, Octylphenyl-salicylat, Dibenzoylresorcin, Bis-(4-tert.butylbenzoyl)-resorcin, Benzoylresorcin 3,5-Di-tert.butyl-4-hydroxybenzoesäure-2,4-di-tert.butylphenylester, 3,5-Di-tert.butyl-4-hydroxybenzoesäurehexadecylester.
   2.4. Acrylate, wie z.B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.
   2.5. Nickelverbindungen, wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert.butylbenzylphosphonsäure-monoalkylestern, wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.
   2.6. Sterisch behinderte Amine, wie z.B. Bis-(2,2,6,6-tetramethyl-piperidyl)-sebacat, Bis-(1,2,2,6,6-pentamethylpiperidyl)-sebacat, n-Buty-3,5-di-tert.butyl-4-hydroxybenzylmalonsäure-bis(1,2, 2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-Bis-(2,2,6,6-Tetramethyl-4-piperidyl)-hexamethylendiamin und 4-tert.Octylamino-2,6-dichlor-1,3,5-s-triazin, Tetrakis-(2,2,6,6-tetramethyl-4-piperidyl)-1,2,3,4-butantetraoat, 1,1'-(1,2-Ethandiyl)-bis-(3,3,5,5-tetramethyl-piperazinon), Bis-(2,2,6,6-tetramethylpiperidyl)-succinat, Bis-(1-octyloxy-2,2,6,6-tetramethylpiperidyl)-sebacat.
   2.7. Oxalsäurediamide, wie z.B. 4,4'-Di-octyloxy-oxanilid, 2,2'-Di-octyloxy-5,5'-di-tert.butyl-oxanilid, 2,2'-Di-dodecycloxy-5,5'-tert.butyl-oxanilid, 2-Ethoxy-2'-ethyl-oxanilid, N,N'-Bis-(3-dimethylaminopropyl)-oxalamid, 2-Ethoxy-5-tert.butyl-2'-ethyloxanilid und dessen Gemisch mit 2-Ethoxy-2'-ethyl-5,4'-di-tert.butyl-oxanilid, Gemische von o- und p-Methoxy- sowie von o- und p-Ethoxy-di-substituierten Oxaniliden.
   2.8. 2-(2-Hydroxyphenyl)-1,3,5-triazine, wie z.B. 2,4,6-Tris(2-hydroxy-4-octyloxyphenyl)-1,3,5-triazin, 2-(2-Hydroxy-4-octyloxyphenyl)-4,6-bis-(2,4-dimethylphenyl)-1,3,5-triazin, 2-(2,4-Dihydroxyphenyl)-4,6-bis(2,4-dimethylphenyl)-1,3,5-triazin, 2,4-Bis-(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethylphenyl)-1,3,5-triazin.
3. Metalldesaktivatoren, wie z.B. N,N'-Diphenyloxalsäurediamid, N-Salicylal-N'-salicyloylhydrazin, N,N'-Bis-(salicyloyl)-hydrazin, N,N'-Bis-(3,5-di-tert.butyl-4-hydroxyphenylpropionyl)-hydrazin, 3-Salicyloylamino-1,2,4-triazol, Bis-(benzyliden)-oxalsäuredihydrazid.
4. Phosphite und Phosphonite, wie z.B. Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Bis-(2,4-di-tert.butylphenyl)-pentaerythritdiphosphit, Tristearyl-sorbit-triphosphit, Tetrakis-(2,4-di-tert.butylphenyl)-4,4'-biphenylen-diphosphonit, 3,9-Bis-(2,4-di-tert.butylphenoxy)-2,4,8,10-tetraoxa-3,9-diphosphaspiro[5.5]undecan.
5. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.
6. Polyamidstabilisatoren, wie z.B. Kupfersalze in Kombination mit Jodiden und/oder Phosphorverbindungen und Salze des zweiwertigen Mangans.
7. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.
8. Nukleierungsmittel, wie z.B. 4-tert.Butylbenzoesäure, Adipinsäure, Diphenylessigsäure.
9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Russ, Graphit.
10. Sonstige Zusätze, wie z.B. Weichmacher, Gleitmittel, Emulgatoren, Pigmente, Optische Aufheller, Flammschutzmittel, Antistatika, Treibmittel.

Von besonderer Bedeutung ist der Zusatz von sterisch gehinderten Aminen (Abschnitt 2.6 der obigen Aufzählung), da diese mit den modifizierten Polymeren eine besonders wirksame Lichtstabilisierung ergeben.

Sofern die Zusätze Stabilisatoren sind, werden diese vorzugsweise in einer Menge von insgesamt 0,05 bis 5 Gew.-% zugesetzt.

Eine weitere Ausgestaltung der Erfindung ist, dass man zusätzlich zu einer Verbindung der Formel I einen weiteren Stabilisator in das Polymer einbaut. Von besonderem Interesse ist der zusätzliche Einbau eines sterisch gehinderten Amins. Je nachdem, ob man ein sterisch gehindertes Amin verwendet, das eine ethylenisch ungesättigte Gruppe oder eine andere funktionelle Gruppe enthält, kann der Einbau durch Copolymerisation oder Copolykondensation oder Copolyaddition oder durch Reaktion mit einem Polymeren, das geeignete funktionelle Gruppen enthält, geschehen.

Sterisch gehinderte Amine, die ethylenisch ungesättigte Gruppen enthalten und erfindungsgemäss zur Copolymerisation geeignet sind z.B. die im US-A-3 705 166 beschriebenen Acryl- und Methacrylsäurederivate des 2,2,6,6-Tetramethylpiperidins sowie dessen N-Alkyl und N-Alkoxyderivate. Weitere copolymerisierbare Derivate des Tetramethylpiperidins sind im US-A-4 210 612 und in der EP-A- 389,420 beschrieben.

Sterisch gehinderte Amine, die mit funktionellen Polymeren reagieren können, sind z.B. solche, die Hydroxylgruppen enthalten, wie z.B. das 2,2,6,6,-Tetramethylpiperidinol-4, das 1,2,2,6,6-Pentamethylpiperidinol-4, das 1-Hydroxyethyl-2,2,6,6-tetramethylpiperidinol-4 oder die im US-A-4 087 404 und in der EP-A 389,419 beschriebenen Verbindungen; oder solche, die Aminogruppen enthalten, wie z.B. das 4-Amino-2,2,6,6-tetramethylpiperidin oder die im US-A-3 904 581 beschriebenen 4-Aminopiperidine.

Der Einbau solcher sterisch gehinderter Amine kann gleichzeitig mit dem Einbau der Triazine der Formel I oder vorher oder nachher geschehen. Die hierfür benutzten Verfahren sind dieselben wie für den Einbau der Triazine. Nähere Details finden sich in den später folgenden Beispielen.

Wird ausser der Triazinverbindung der Formel I auch ein sterisch gehindertes Amin in das Polymere eingebaut, so verwendet man letzteres vorzugsweise in einer Menge, die 0,1 bis 15 Gew.-% des modifizierten Polymeren entspricht.

Die erfindungsgemäss modifizierten Polymeren können für die üblichen Verwendungsformen von Polymeren verwendet werden, wie z.B. als Formteile, Rohre, Platten, Folien, Fasern, Giessharze, Klebstoffe oder Beschichtungen. Bevorzugt verwendet man sie als Bindemittel für Lacke und zwar sowohl für pigmentierte wie unpigmentierte Lacke.

Weiterhin können die modifizierten Polymeren als Stabilisatoren für organische Materialien, vorwiegend für Polymere, verwendet werden. Hierzu verwendet man vorzugsweise solche modifizierte Polymere, die mindestens 5 % einer Verbindung der Formel I in eingebauter Form enthalten. Diese können zum Stabilisieren von organischen Materialien wie z.B. von Oelen, Fetten, Wachsen, Kosmetika oder photographischen Materialien verwendet werden, insbesondere aber zum Stabilisieren von organischen Polymeren. Beispiele für Polymere, die so stabilisiert werden können, sind die folgenden:
1. Polymere von Mono- und Diolefinen, beispielsweise Polypropylen, Polyisobutylen, Polybuten-1, Polymethylpenten-1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen niederer Dichte (LDPE), lineares Polyethylen niederer Dichte (LLDPE).
2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).
3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymer, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat- Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropyle/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere und LLDPE/Ethylen-Acrylsäure-Copolymere.
3a. Kohlenwasserstoffharze (z.B. C₅-C₉) inklusive hydrierte Modifikationen davon (z.B. Klebrigmacherharze).
4. Polystyrol, Poly-(p-methylstyrol), Poly-(α-methylstyrol).
5. Copolymere von Styrol oder α-Methylstyrol mit Dienen oder Acrylderivaten, wie z.B. Styrol-Butadien, Styrol-Acrylnitril, Styrol-Alkylmethacrylat, Styrol-Butadien-Alkylacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.
6. Pfropfcopolymere von Styrol oder a-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z.B. als sogenannte ABS-, MBS-, ASA- oder AES-Polymere bekannt sind.
7. Halogenhaltige Polymere, wie z.B. Polychloropren, Chlorkautschuk, chloriertes oder chlorsulfoniertes Polyethylen, Copolymere von Ethylen und chloriertem Ethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.
8. Polymere, die sich von α,β-ungesättigten Säuren und deren Derivaten ableiten, wie Polyacrylate und Polymethacrylate, Polyacrylamide und Polyacrylnitrile.
9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.
10. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.
11. Homo- und Copolymere von cyclischen Ethern, wie Polyalkylenglykole, Polyethylenoxyd, Polypropylenoxyd oder deren Copolymere mit Bisglycidylethern.
12. Polyacetale, wie Polyoxymethylen, sowie solche Polyoxymethylene, die Comonomere, wie z.B. Ethylenoxid, enthalten; Polyacetale, die mit termoplastischen Polyurethanen, Acrylaten oder MBS modifiziert sind.
13. Polyphenylenoxide und -sulfide und deren Mischungen mit Styrolpolymeren oder Polyamiden.
14. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.
15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, Polyamid 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid, Poly-m-phenylen-isophthalamid. Block-Copolymere der vorstehend genannten Polyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").
16. Polyharnstoffe, Polyimide, Polyamid-imide und Polybenzimidazole.
17. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.
18. Polycarbonate und Polyestercarbonate.
19. Polysulfone, Polyethersulfone und Polyetherketone.
20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.
21. Trocknende und nicht-trocknende Alkydharze.
22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.
23. Vernetzbare Acrylharze, die sich von substituierten Acrylsäureestern ableiten, wie z.B. von Epoxyacrylaten, Urethan-acrylaten oder Polyester-acrylaten.
24. Alkydharze, Polyesterharze und Acrylatharze, die mit Melaminharzen, Harnstoffharzen, Polyisocyanaten oder Epoxidharzen vernetzt sind.
25. Vernetzte Epoxidharze, die sich von Polyepoxiden ableiten, z.B. von Bis-glycidylethern oder von cycloaliphatischen Diepoxiden.
26. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.
27. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO.

Die meisten Verbindungen der Formel I sind bekannte Verbindungen, die z.B. im US-A-3 118 887, US-A-3 244 708 oder in der US-Anmeldung No. 446 369 beschrieben sind.

Die folgenden Beispiele erläutern die Erfindung näher. Darin bedeuten Teile Gewichtsteile und % Gewichts-%, M̅ₙ Zahlenmittel des Molekulargewichtes und M̅_{w} Gewichtsmittel des Molekulargewichtes.

### Beispiel 1 - Einbau durch polymeranaloge Reaktion

### a) Herstellung eines funktionellen Polymeren

In einem 6 l-Glaskolben werden unter Stickstoff 2070 ml trockenes Toluol zum Rückfluss erwärmt. Unter Rühren lässt man dazu gleichzeitig die Lösungen I und II zutropfen.
- Lösung I:: 606,8 g Methylmethacrylat
1212,3 g Butylmethacrylat
631,5 g Glycidylmethacrylat
- Lösung II:: 60 g Azoisobuyronitril
750 ml Toluol
Lösung I wird innerhalb 3 h zugetropft, Lösung II innerhalb 3,5 h. Anschliessend wird noch 1 h zum Rückfluss erwärmt. Man erhält eine viskose ca. 50%ige Polymerlösung, die klar und farblos ist. Die Lösung enthält 4,33 Mol Epoxidgruppen.

### b) Umsetzung mit einem Triazinderivat

In einem 750 ml Glaskolben werden 275 g der obigen Polymerlösung mit 4,5 g 2,4-Di-(2,4-dimethylphenyl)-6-(2,4-dihydroxyphenyl)-1,3,5-triazin gemischt und 48 h zum Rückluss erwärmt (Innentemperatur ca. 110°C). Hierbei reagieren die Epoxidgruppen des Polymeren aus Beispiel 1a mit den Hydroxylgruppen der Triazinverbindung. Man erhält eine hellgelbe Klare Lösung des modifizierten Polymeren. Das Polymer enthält gemäss Elementaranalyse 3,2 % der Triazinverbindung. Gemäss Gelpermationschromatographie beträgt das Molekulargewicht M̅ₙ 4363 und M̅_{w} 13308.

### c) Prüfung der Witterungsbeständigkeit

Die so erhaltene Lösung des modifizierten Acrylatharzes wird im Festkörper-Verhältnis 65:35 mit einem Melaminharz (Cymel® 327, Cyanamid Corp.) gemischt. Dazu werden 2,5 % (bezogen auf Festkörper) eines Verlaufsmittels (Baysilon® A, Bayer AG) (1%ige Lösung in Xylol) zugesetzt.

Diese Klarlackformulierung wird mit Xylol/Butanol/Butylglykolacetat 13:6:1 auf Spritzfähigkeit verdünnt, auf ein mit einem Silbermetallic Basislack lackiertes Aluminiumblech aufgetragen und bei 130°C 30 Minuten eingebrannt. Es resultiert eine Filmdicke von 40-45 µm.
Als Vergleich dient der unter a) beschriebene Klarlack, der kein Triazinderivat enthält. Die Witterungsbeständigkeit der Proben wird in einem UVCON®-Bewitterungsgerät UVB-313 bei einem Cyklus von 4 h UV-Bestrahlung bei 60°C und 4 h Kondensation bei 50°C geprüft.
Gemessen wird der 20°-Glanz nach DIN 67530 vor der Bewitterung und nach 400 h Bewitterung.

| Eingebauter Stabilisator | 20°-Glanz (%) nach | |
|---|---|---|
| | 0 | 400 h |
| Keiner | 81 | 1 |
| 2,1 % Triazin | 83 | 61 |

### Beispiel 2 - Einbau durch Copolymerisation

A) In einem 750 ml-Glaskolben werden 200 ml trockenes Xylol auf 135°C erwärmt. Unter Rühren lässt man dazu gleichzeitig innerhalb 3 g die Lösungen III und IV zutropfen.
   - Lösung III:: 75 g Butylacrylat
   81 g Butylmethacrylat
   90 g Hydroxyethylacrylat
   45 g Styrol
   9 g Eisessig
   18,6 g t-Amylperbenzoat
   - Lösung IV:: 40 ml Xylol
   10,5 g 1-Methacryloyloxy-2-hydroxy-3-[4-(4,6-di[2,4-dimethylphenyl]-3-triazin-2-yl)-3-hydroxyphenoxy]-propan der Formel 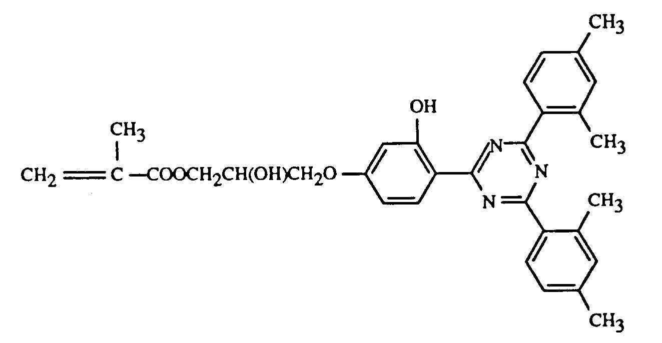
   Nach der Zugabe erwärmt man noch 1 h auf 135°C und erhält eine hellgelbe viskose Lösung des Copolymeren. Das Copolymer enthält gemäss Elementaranalyse 3,3 % der obigen Triazinverbindung und hat ein Molekulargewicht M̅ₙ 3248 und M̅_{w} 8135.
B) Die so erhaltene Harz-Lösung wird im Festkörperverhältnis 65:35 mit einem Melaminharz (Cymel® 303, Cyanamid Corp.) gemischt. Dazu werden als Härtungskatalysator 0,5 % (bezogen auf Festkörper) p-Toluolsulfonsäure gegeben.

Diese Klarlackformulierung wird mit Xylol auf Spritzfähigkeit verdünnt und auf ein mit einem Silbermetallic-Basislack lackiertes Aluminiumblech aufgetragen. Nach dem Einbrennen (30 Minuten bei 120°C) resultiert eine Filmdicke von 40-45 µm. Als Vergleich dient ein Klarlack, der kein Triazinderivat enthält und durch Polymerisation der Lösung III und Abmischung mit Melaminharz erhalten wurde. Die Proben werden im UVCON®-Gerät auf ihre Witterungsbeständigkeit geprüft.

| Eingebauter Stabilisator | 20°-Glanz (%) nach | | | | |
|---|---|---|---|---|---|
| | 0 | 400 | 1600 | 2400 | 3200 h |
| Keiner | 90 | 83 | 18 | - | - |
| 2,1 % Triazin | 93 | 90 | 90 | 92 | 91 |

### Beispiel 3 - Gleichzeitiger Einbau eines gehinderten Amins (HALS)

Es wird wie in Beispiel 2A) gearbeitet, jedoch werden der Lösung IV noch 5,1 g 4-Acryloyloxy-1,2,2,6,6-pentamethylpiperidin zugegeben. Man erhält eine hellgelbe viskose Lösung des Copolymeren. Das Copolymere enthält 3,3 % des Triazinderivates und 1,6 % des Piperidinderivates und hat ein Molekulargewicht M̅ₙ 3063 und M̅_{w} 9283.

100 Teile der Harz-Lösung werden mit 0,14 Teilen Zinkoctoat, 30 Teilen eines trimerisierten Diisocyanates (Desmodur® 3390) und 1,6 Teilen eines Verlaufhilfsmittels (Byk® 300) vermischt.

In derselben Weise werden 100 Teile der Harz-Lösung von Beispiel 2A mit Zinkoctoat, Desmodur® 3390 und Byk® 300 gemischt.

Als Vergleich werden 100 Teile der aus Lösung III des Beispiels 2A erhaltenen Polymerlösung (die kein Triazin enthält) in derselben Weise mit Zinkoctoat, Desmodur® 3390 und Byk® 300 gemischt.

Alle drei Harzlösungen werden mit Xylol auf Spritzfähigkeit verdünnt und auf ein mit einem Silbermetallic-Basislack grundiertes Aluminiumblech aufgetragen. Die Proben werden 20 min bei 140°C eingebrannt. Die resultierende Filmdicke ist 45-50 µm.

Die Witterungsbeständigkeit der Proben wird im UVCON® 313 bei einem Cyklus von 8 h UV-Bestrahlung bei 70°C und 4 h Kondensation bei 50°C geprüft und der 20°-Glanz nach DIN 67530 gemessen.

| Eingebauter Stabilisator | 20°-Glanz (%) nach | | | | |
|---|---|---|---|---|---|
| | 1600 | 2800 | 4000 | 6000 | 7600 h |
| Keiner | 52 | 36 ^{*)} | | | |
| 2,2 % Triazin | 84 | 78 | 74 | 58 ^{*)} | |
| 2,2 % Triazin + 1,1 % HALS | 89 | 83 | 81 | 63 | 52 |

| | | | | | |
|---|---|---|---|---|---|
| ^{*)} Rissbildung | | | | | |

### Beispiel 4 - Einbau in ein UV-härtbares System

Ein UV-härtbares System wird bereitet aus
66 Teilen eines Urethanacrylates (Genomer® T 1600, Fa. Mäder),
32 Teilen 1,6-Hexandiol-diacrylat (Sartomer® SR 238) und
2 Teilen 1-Benzoylcyclohexanol (als Initiator).

Ein Teil wird mit 1,5 % des in Beispiel 2 beschriebenen ungesättigten Triazinderivates versetzt. Ein zweiter Teil wird mit 1,5 % dieses Triazinderivates und 0,5 % 4-Acryloyloxy-1-octyloxy-2,2,6,6-tetramethylpiperidin (HALS) versetzt. Ein dritter Teil wird ohne Stabilisatorzusatz gehärtet.

Die Formulierungen werden auf ein mit einem Silbermetallic-Lack grundiertes Aluminiumblech aufgebracht und in einem UV-Prozessor der Fa. PPG mit 2 Lampen à 80 W/cm in zwei Durchläufen bei 10 m/min Durchlaufgeschwindigkeit gehärtet. Es resultiert eine Filmdicke von ca. 40 µm.

Die Probenbleche werden in einem UVCON® 313 bei einem Cyklus von 4 h UV-Bestrahlung bei 60°C und 4 h Kondensation bei 50°C bewittert und der 20°-Glanz nach DIN 67530 gemessen.

| Eingebauter Stabilisator | 20°-Glanz (%) nach | | | | |
|---|---|---|---|---|---|
| | 0 | 800 | 1600 | 2800 | 4000 h |
| Keiner | 81 | 21 ^{*)} | | | |
| 1,5 % Triazin | 80 | 88 | 85 | 68 ^{*)} | |
| 1,5 % Triazin + 0,5 % HALS | 82 | 88 | 88 | 87 | 82 |

| | | | | | |
|---|---|---|---|---|---|
| ^{*)} Rissbildung | | | | | |

### Beispiel 5 - Einbau in ein strahlenhärtbares System

Ein strahlenhärtbarer Klarlack wird bereitet auf 70 Teilen eines aliphatisches Urethanacrylates (Ebecryl® 284, Fa. UCB) und 30 Teilen 1,6-Hexandiol-diacrylat. Ein Teil davon wird mit 2 % des in Beispiel 2 beschriebenen ungesättigten Triazinderivates (gelöst in Xylol) versetzt.

Die Lackproben werden auf ein weiss-grundiertes Aluminiumblech aufgebracht und in einen Elektronenstrahlgerät mit einer Dosis von 1 Mrad bestrahlt. Die resultierende Filmdicke beträgt ca. 40 µm.

Um den Einbau des Triazins zu kontrollieren wird ein Teil des Probenbleches 2 h mit Toluol extrahiert und das Extrakt analytisch untersucht. Die Konzentration an extrahiertem Triazin ist kleiner als 0,1 %. Das beweist den quantitativen Einbau des Triazins in den Lack.

Die Proben werden im UVCON® 313 wie in Beispiel 4 bewittert und der 20°-Glanz gemessen.

| Eingebauter Stabilisator | 20°-Glanz (%) nach | | | | |
|---|---|---|---|---|---|
| | 0 | 400 | 800 | 1600 | 2400 h |
| Keiner | 81 | 78 | 79 ^{*)} | | |
| 2,0 % Triazin | 83 | 81 | 82 | 78 | 72 |

| | | | | | |
|---|---|---|---|---|---|
| ^{*)} Delaminierung | | | | | |

## Patentansprüche

1. Verfahren zum Einbau von o-Hydroxyphenyl-s-triazinen in organische Polymere, dadurch gekennzeichnet, dass man eine Verbindung der Formel I, 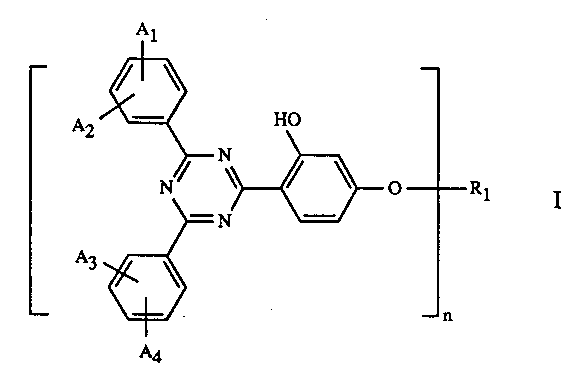 worin n 1 oder 2 ist,
A₁, A₂, A₃ und A₄ unabhängig voneinander Wasserstoff, C₁-C₁₂-Alkyl, Cyclohexyl oder Halogen bedeuten,
R₁, wenn n 1 ist, Wasserstoff, durch OH, -COOH, -COOR₂, -NHR₃, -CONHR₄, 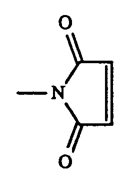 oder/und -O-CO-R₄ substituiertes C₁-C₁₈-Alkyl, durch OH substituiertes und durch ein oder mehrere O unterbrochenes C₄-C₂₀-Alkyl, durch OH und C₁-C₁₂-Alkoxy oder Phenoxy substituertes C₂-C₄-Alkyl, durch OH oder -OCOR₄ substituiertes Cyclohexyl, C₂-C₆-Alkenyl, Glycidyl oder eine der Gruppen 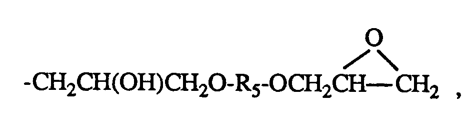 -CO-R₆-COOH oder -CO-NH-R₇-NCO bedeutet,
und wenn n 2 ist, eine der Gruppen -CH₂CH(OH)CH₂-, -CO-CH=CH-CO-,
-CH₂CH(OH)CH₂O-R₅-OCH₂CH(OH)CH₂- oder
-CH₂CH(R₈)O-CO-CH=CH-CO-OCH(R₈)CH₂- bedeutet,
R₂ C₁-C₄-Alkyl, Glycidyl oder C₃-C₅-Alkenyl bedeutet,
R₃ Wasserstoff, C₁-C₁₂-Alkyl, C₃-C₅-Alkenyl oder Cyclohexyl bedeutet,
R₄ C₂-C₆-Alkenyl oder C₂-C₆-Hydroxyalkyl bedeutet,
R₅ C₂-C₁₀-Alkylen, Phenylen oder eine Gruppe 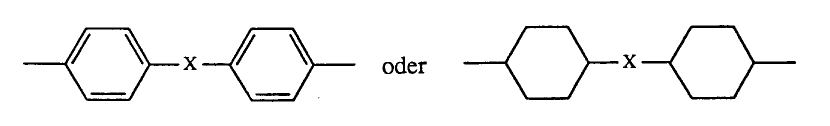 bedeutet, worin X -O-, -S-, -SO₂, -CH₂- oder -C(CH₃)₂- ist, oder R₅ eine Gruppe -CO-R₉-CO- bedeutet,
R₆ C₂-C₁₄-Alkylen, -CH=CH- oder o-Phenylen bedeutet,
R₇ C₂-C₁₀-Alkylen, Phenylen, Tolylen oder eine Gruppe der Formel 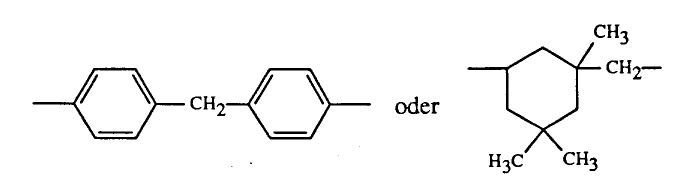 bedeutet,
R₈ Wasserstoff oder Methyl bedeutet und
R₉ C₂-C₁₀-Alkylen, -CH=CH- oder Phenylen bedeutet,
entweder bei der Herstellung des Polymeren durch Copolymerisation, Copolykondensation oder Copolyaddition einbaut oder mit einem Polymeren, das geeignete funktionelle Gruppen enthält, reagieren lässt.

2. Verfahren gemäss Anspruch 1, dadurch gekennzeichnet, dass man eine Verbindung der Formel I einbaut,
worin n 1 oder 2 ist,
A₁, A₂, A₃ und A₄ Wasserstoff, C₁-C₄-Alkyl oder Chlor bedeuten,
R₁, wenn n 1 ist, Wasserstoff, durch OH, -COOH, -COOR₂, -NHR₃, -CONHR₄ oder/und -O-CO-R₄ substituiertes C₁-C₄-Alkyl, durch OH substituiertes und durch ein oder mehrere O unterbrochenes C₄-C₂₀-Alkyl, durch OH und C₁-C₁₂-Alkoxy oder Phenoxy substituertes Propyl, durch OH oder -OCOR₄ substituiertes Cyclohexyl, Allyl, Glycidyl oder eine der Gruppen 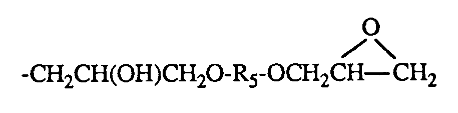 oder -CO-NH-R₇-NCO bedeutet,
und wenn n 2 ist, eine der Gruppen -CH₂CH(OH)CH₂-,
-CH₂CH(OH)CH₂O-R₅-OCH₂CH(OH)CH₂- oder
-CH₂CH(R₈)O-CO-CH=CH-CO-OCH(R₈)CH₂- bedeutet,
R₂ C₁-C₄-Alkyl, Glycidyl oder Allyl bedeutet,
R₃ C₁-C₁₂-Alkyl bedeutet,
R₄ C₂-C₆-Alkenyl bedeutet,
R₅ C₂-C₁₀-Alkylen, Phenylen oder eine Gruppe 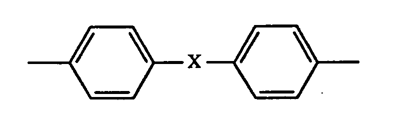 bedeutet, worin X -O-, -S-, -SO₂, -CH₂- oder -C(CH₃)₂- ist, oder R₅ eine Gruppe -CO-R₉-CO- bedeutet,
R₇ C₂-C₁₀-Alkylen, Phenylen, Tolylen oder eine Gruppe der Formel 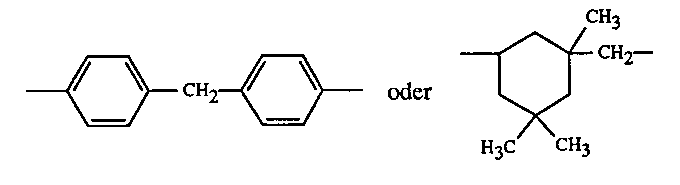 bedeutet,
R₈ Wasserstoff oder Methyl bedeutet und
R₉ C₂-C₁₀-Alkylen, -CH=CH- oder Phenylen bedeutet.

3. Verfahren gemäss Anspruch 1, dadurch gekennzeichnet, dass man eine Verbindung der Formel I einbaut, worin n 1 oder 2 ist,
A₁ und A₃ Wasserstoff, Methyl oder Chlor bedeuten,
A₂ und A₄ Wasserstoff oder Methyl bedeuten,
R₁, wenn n 1 ist, Wasserstoff, durch OH, -COOH, -COOR₂ oder/und -O-CO-R₄ substituiertes C₁-C₄-Alkyl, durch OH substituiertes und durch ein oder mehrere O unterbrochenes C₄-C₂₀-Alkyl, durch OH und C₄-C₁₂-Alkoxy substituiertes Propyl, Glycidyl, Allyl oder eine Gruppe 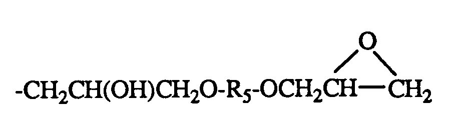 bedeutet, und wenn n 2 ist, eine der Gruppen -CH₂CH(OH)CH₂- oder
-CH₂CH(OH)CH₂O-R₅-OCH₂CH(OH)CH₂- bedeutet,
R₂ C₁-C₄-Alkyl oder Allyl bedeutet,
R₄ C₂-C₄-Alkenyl bedeutet und
R₅ C₄-C₈-Alkylen oder 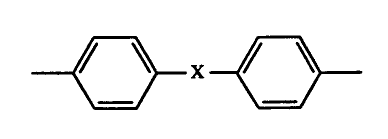 bedeutet, worin X -CH₂- oder -C(CH₃)₂- ist.

4. Verfahren gemäss Anspruch 1, dadurch gekennzeichnet, dass man eine Verbindung der Formel I, worin n 1 oder 2 ist, R₁, wenn n 1 ist, C₂-C₆-Alkenyl, durch -COOR₂, -NHR₃, -CONHR₄ oder -O-CO-R₄ substituiertes C₁-C₁₈-Alkyl, durch -O-CO-R₄ substituiertes Cyclohexyl oder eine Gruppe -CO-CH=CH-COOH bedeutet, und wenn n 2 ist, eine Gruppe -CO-CH=CH-CO-, -CH₂CH(R₈)O-CO-CH=CH-CO-OCH(R₈)CH₂- oder -CH₂CH(OH)CH₂O-CO-CH=CH-CO-OCH₂CH(OH)CH₂- bedeutet, R₂ C₃-C₅-Alkenyl ist, R₃ Allyl ist, R₄ C₂-C₆-Alkenyl ist und R₈ Wasserstoff oder Methyl ist, mit einem oder mehreren ethylenisch ungesättigten Monomeren copolymerisiert.

5. Verfahren gemäss Anspruch 4, dadurch gekennzeichnet, dass man als Monomere Acrylsäure, Methacrylsäure, Ester oder Amide der Acryl- oder Methacrylsäure, Styrol oder Acrylnitril verwendet.

6. Verfahren gemäss Anspruch 1, dadurch gekennzeichnet, dass man eine Verbindung der Formel I, worin n 1 oder 2 ist, R₁, wenn n 1 ist, durch OH und -COOH, -COOR₂ oder -NHR₃ substituiertes C₁-C₁₈-Alkyl oder eine Gruppe -CH₂CH(OH)CH₂OH oder 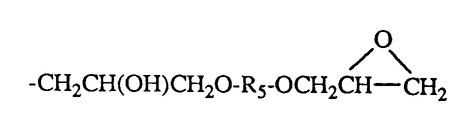 bedeutet, und wenn n 2 ist, eine Gruppe -CH₂CH(OH)CH₂O-R₅-OCH₂CH(OH)CH₂- bedeutet, R₂ C₁-C₄-Alkyl bedeutet, R₃ Wasserstoff, C₁-C₁₂-Alkyl, Allyl oder Cyclohexyl bedeutet und R₅ C₂-C₁₀-Alkylen, Phenylen oder eine Gruppe 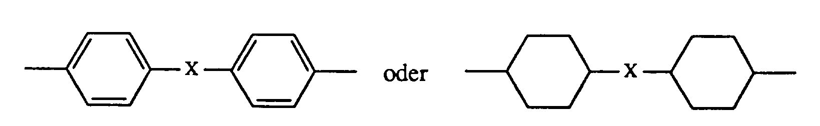 bedeutet, worin X -O-, -S-, -SO₂, -CH₂- oder -C(CH₃)₂- ist,
durch Copolykondensation oder Copolyaddition in einen Polyester oder Polyetherester, ein Polyamid, Polyurethan, Polycarbonat, Epoxidharz, Phenolharz, Melaminharz oder Alkydharz einbaut.

7. Verfahren gemäss Anspruch 1, dadurch gekennzeichnet, dass man ein Polymer, das Hydroxyl-, Carboxyl-, Anhydrid-, Amino-, Epoxid- oder Isocyanatgruppen enthält, mit einer Verbindung der Formel I umsetzt, die solche funktionelle Gruppen enthält, die mit den funktionellen Gruppen des Polymeren reagieren können.

8. Verfahren gemäss Anspruch 1, dadurch gekennzeichnet, dass man eine ethylenisch ungesättigte Verbindung der Formel I, wie sie in Anspruch 4 definiert ist, auf ein Kohlenwasserstoffpolymer aufpfropft.

9. Ein nach dem Verfahren des Anspruches 1 modifiziertes Polymer, enthaltend 0,05 bis 50 Gew.-% einer Triazinverbindung der Formel I chemisch gebunden an das Polymer.

10. Modifiziertes Polymer gemäss Anspruch 9, enthaltend als physikalischen Zusatz mindestens einen Stabilisator, ein Verarbeitungshilfsmittel, ein Pigment oder einen sonstigen Zusatz.

11. Modifiziertes Polymer gemäss Anspruch 9, enthaltend ein sterisch gehindertes Amin in das Polymer eingebaut.

12. Verwendung eines modifizierten Polymeren gemäss Anspruch 9 als Bindemittel für Lacke.

## Claims

1. A process for incorporating an o-hydroxyphenyl-s-triazine in an organic polymer, which process comprises either incorporating a compound of formula I 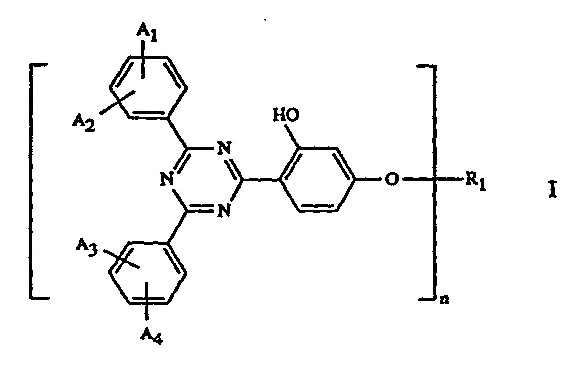 wherein n is 1 or 2,
A₁, A₂, A₃ and A₄ are independently of one another hydrogen, C₁-C₁₂alkyl, cyclohexyl or halogen,
R₁, when n is 1, is hydrogen, C₁-C₁₈alkyl which is substituted by OH, -COOH, -COOR₂, -NHR₃, -CONHR₄, 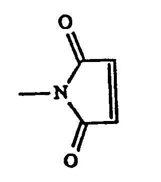 and/or -O-CO-R₄, C₄-C₂₀alkyl which is substituted by OH and interrupted by one or more O, C₂-C₄alkyl which is substituted by OH and C₁-C₁₂alkoxy or phenoxy, cyclohexyl which is substituted by OH or -OCOR₄, or is C₂-C₆alkenyl, glycidyl or one of the groups 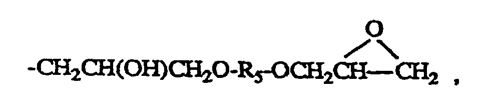 -CO-R₆-COOH or -CO-NH-R₇-NCO
and, when n is 2, is one of the groups -CH₂CH(OH)CH₂-, -CO-CH=CH-CO-, -CH₂CH(OH)CH₂O-R₅-OCH₂CH(OH)CH₂- or -CH₂CH(R₈)O-CO-CH=CH-CO-OCH(R₈)CH₂-, R₂ is C₁-C₄alkyl, glycidyl or C₃-C₅alkenyl,
R₃ is hydrogen, C₁-C₁₂alkyl, C₃-C₅alkenyl or cyclohexyl,
R₄ is C₂-C₆alkenyl or C₂-C₆hydroxyalkyl,
R₅ is C₂-C₁₀alkylene, phenylene or a group 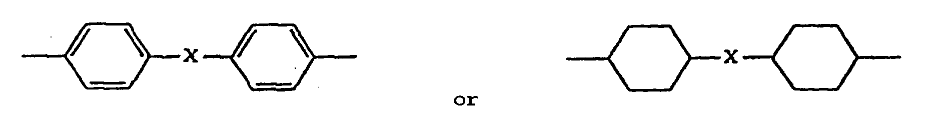 wherein X is -O-, -S-, -SO₂-, -CH₂- or -C(CH₃)₂-, or R₅ is a group -CO-R₉-CO-,
R₆ is C₂-C₁₄alkylene, -CH=CH- or o-phenylene,
R₇ is C₂-C₁₀alkylene, phenylene, tolylene or a group of formula 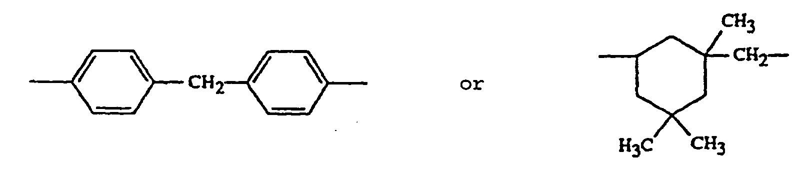 R₈ is hydrogen or methyl, and
R₉ is C₂-C₁₀alkylene, -CH=CH- or phenylene,
during the preparation of the polymer by copolymerisation,
copolycondensation or copolyaddition, or reacting it with a polymer which contains suitable functional groups.

2. A process according to claim 1, which comprises incorporating a compound of formula I wherein n is 1 or 2,
A₁, A₂, A₃ and A₄ are hydrogen, C₁-C₄alkyl or chloro,
R₁, when n is 1, is hydrogen, C₁-C₄alkyl which is substituted by OH, -COOH, -COOR₂, -NHR₃, -CONHR₄ and/or -O-CO-R₄, C₄-C₂₀alkyl which is substituted by OH and interrupted by one or more O, propyl which is substituted by OH and C₁-C₁₂alkoxy or phenoxy, cyclohexyl which is substituted by OH or -OCOR₄, or is allyl, glycidyl or one of the groups 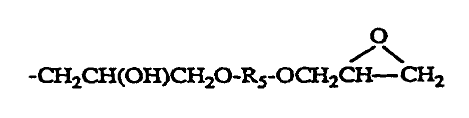 or -CO-NH-R₇-NCO, and, when n is 2,
is one of the groups -CH₂CH(OH)CH₂-, -CH₂CH(OH)CH₂O-R₅-OCH₂CH(OH)CH₂- or -CH₂CH(R₈)O-CO-CH=CH-CO-OCH(R₈)CH₂-,
R₂ is C₁-C₄alkyl, glycidyl or allyl,
R₃ is C₁-C₁₂alkyl,
R₄ is C₂-C₆alkenyl,
R₅ is C₂-C₁₀alkylene, phenylene or a
group 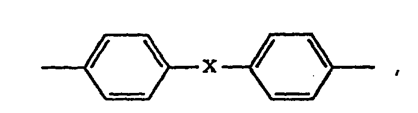 wherein X is -O-, -S-, -SO₂-, -CH₂- or -C(CH₃)₂, or R₅ is a group -CO-R₉-CO-,
R₇ is C₂-C₁₀alkylene, phenylene, tolylene or a group of formula 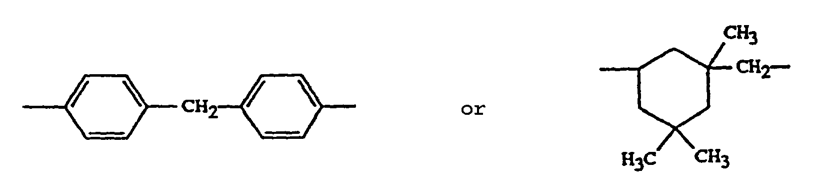 R₈ is hydrogen or methyl, and
R₉ is C₂-C₁₀alkylene, -CH=CH- or phenylene.

3. A process according to claim 1, which comprises incorporating a compound of formula I wherein n is 1 or 2,
A₁ and A₃ are hydrogen, methyl or chloro,
A₂ and A₄ are hydrogen or methyl,
R₁, when n is 1, is hydrogen, C₁-C₄alkyl which is substituted by OH, -COOH, -COOR₂ and/or -O-CO-R₄, C₄-C₂₀alkyl which is substituted by OH and interrupted by one or more O, propyl which is substituted by OH and C₄-C₁₂alkoxy, or is glycidyl, allyl, or a 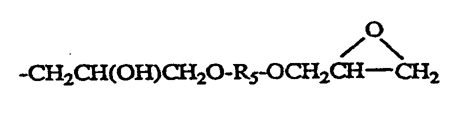 group, and, when n is 2, is one of the groups -CH₂CH(OH)CH₂- or -CH₂CH(OH)CH₂O-R₅-OCH₂CH(OH)CH₂-,
R₂ is C₁-C₄alkyl or allyl,
R₄ is C₂-C₄alkenyl, and
R₅ is C₄-C₈alkylene or 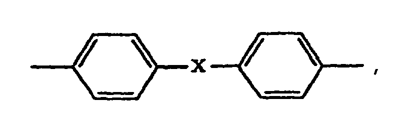 wherein X is -CH₂- or -C(CH₃)₂-.

4. A process according to claim 1, which comprises copolymerising a compound of formula I wherein n is 1 or 2, R₁, when n is 1, is C₂-C₆alkenyl, C₁-C₁₈alkyl which is substituted by -COOR₂, -NHR₃, -CONHR₄ or -O-CO-R₄, cyclohexyl which is substituted by -O-CO-R₄, or is a -CO-CH=CH-COOH group, and, when n is 2, is a group -CO-CH=CH-CO-, -CH₂CH(R₈)O-CO-CH=CH-CO-OCH(R₈)CH₂- or -CH₂CH(OH)CH₂O-CO-CH=CH-CO-OCH₂CH(CH)CH₂-, R₂ is C₃-C₅alkenyl, R₃ is allyl,
R₄ is C₂-C₆alkenyl, and R₈ is hydrogen or methyl, with one or more ethylenically unsaturated monomers.

5. A process according to claim 4, wherein the monomers used are acrylic acid, methacrylic acid, esters or amides of acrylic or methacrylic acid, styrene or acrylonitrile.

6. A process according to claim 1, which comprises incorporating a compound of formula I wherein n is 1 or 2, R₁, when n is 1, is C₁-C₁₈alkyl which is substituted by OH and -COOH, -COOR₂ or -NHR₃, or is a -CH₂CH(OH)CH₂OH or 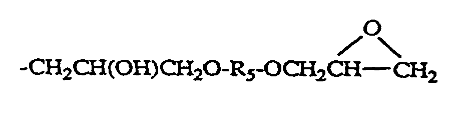 group, and, when n is 2, is a group -CH₂CH(OH)CH₂O-R₅-OCH₂CH(OH)CH₂-, R₂ is C₁-C₄alkyl, R₃ is hydrogen, C₁-C₁₂alkyl, allyl or cyclohexyl, and R₅ is C₂-C₁₀alkylene, phenylene or a group 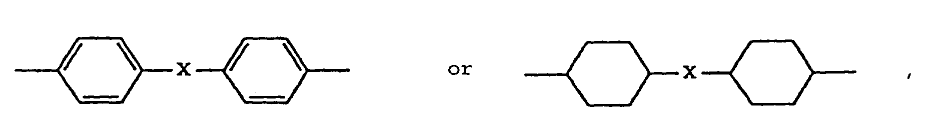 wherein X is -O-, -S-, -SO₂-, -CH₂- or -CH(CH₃)₂-, by copolycondensation or copolyaddition, in a polyester or polyether ester, a polyamide, polyurethane, polycarbonate, epoxy resin, phenolic resin, melamine resin or alkyd resin.

7. A process according to claim 1, which comprises reacting a polymer which contains hydroxyl, carboxyl, anhydride, amino, epoxy or isocyanato groups with a compound of formula I which contains those functional groups which are able to react with the functional groups of the polymer.

8. A process according to claim 1, which comprises grafting an ethylenically unsaturated compound of formula I as defined in claim 4 on to a hydrocarbon polymer.

9. A polymer modified by a process of claim 1 and containing 0.05 to 50 % by weight of a triazine compound of formula I chemically bonded to the polymer.

10. A modified polymer according to claim 9, which contains at least one stabiliser, a processing auxiliary, a pigment or another additive as physical additive.

11. A modified polymer according to claim 9, which contains a sterically hindered amine incorporated in the polymer.

12. Use of a modified polymer according to claim 9 as binder for coating materials.

## Revendications

1. Procédé d'incorporation avec liaison chimique de o-hydroxyphényl-s-triazines dans des matières polymères organiques, procédé caractérisé en ce que l'on incorpore un composé de formule I 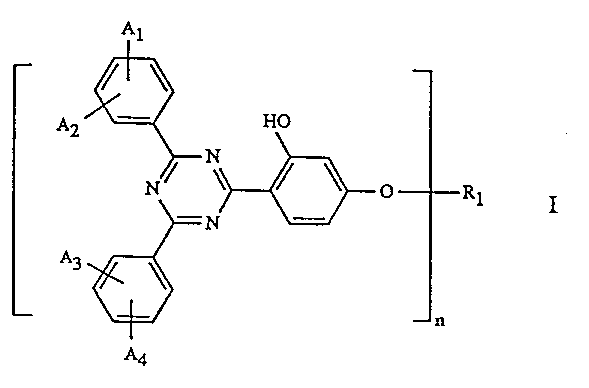 dans laquelle n est le nombre 1 ou 2,
A₁, A₂, A₃ et A₄ représentent chacun, indépendamment les uns des autres, l'hydrogène, un alkyle en C₁-C₁₂, un cyclohexyle ou un halogène,
R₁, si n vaut 1, est l'hydrogène, un alkyle en C₁-C₁₈ substitué par les OH, -COOH, -COOR₂, -NHR₃, -CONHR₄, 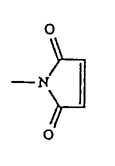 ou/et -O-CO-R₄ , un alkyle en C₄-C₂₀ substitué par OH et interrompu par un ou plusieurs atomes d'oxygène, un alkyle en C₂-C₄ substitué par OH et des alcoxy en C₁-C₁₂ ou phénoxy, un cyclohexyle substitué par des OH ou -OCOR₄, un alcényle en C₂-C₆, un glycidyle ou encore l'un des groupes 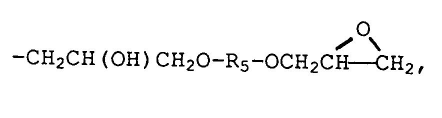 -CO-R₆-COOH ou
-CO-NH-R₇-NCO,
et si n vaut 2, R₁ est l'un des groupes -CH₂CH(OH)CH₂-, -CO-CH=CH-CO-,
-CH₂CH(OH)CH₂O-R₅-OCH₂CH(OH)CH₂- ou -CH₂CH(R₈)O-CO-CH=CH-CO-OCH(R₈)CH₂-,
R₂ désigne un alkyle en C₁-C₄, un glycidyle ou un alcényle en C₃-C₅,
R₃ l'hydrogène, un alkyle en C₁-C₁₂, un alcényle en C₃-C₅ ou un cyclohexyle,
R₄ un alcényle en C₂-C₆ ou un hydroxyalkyle en C₂-C₆,
R₅ un alkylène en C₂-C₁₀, un phénylène ou un groupe 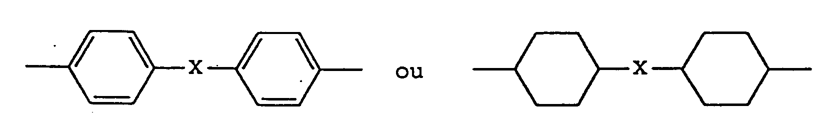 dans lesquels X représente -O-, -S-, -SO₂, -CH₂- ou -C(CH₃)₂-, ou bien R₅ est un groupe -CO-R₉-CO-,
R₆ désigne un alkylène en C₂-C₁₄, un -CH=CH- ou un o-phénylène,
R₇ un alkylène en C₂-C₁₀, un phénylène, un tolylène ou un groupe de formule 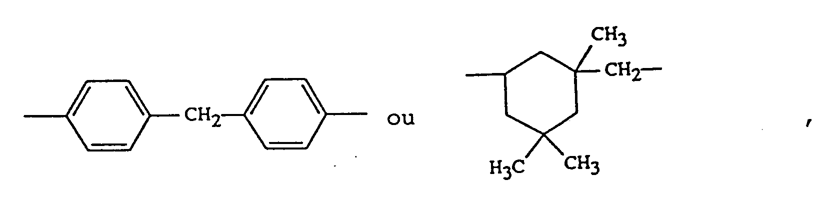 R₈ l'hydrogène ou le méthyle et
R₉ un alkylène en C₂-C₁₀, un -CH=CH- ou un phénylène, lors de la préparation du polymère par copolymérisation, copolycondensation ou copolyaddition, ou bien on fait réagir ce composé avec un polymère contenant les groupes fonctionnels appropriés.

2. Procédé selon la revendication 1, caractérisé en ce que l'on incorpore un composé de formule I,
dans laquelle n vaut 1 ou 2,
A₁, A₂, A₃ et A₄ sont l'hydrogène, des alkyles en C₁-C₄ ou le chlore,
R₁, si n vaut 1, est l'hydrogène, un alkyle en C₁-C₄ avec comme substituants des OH, -COOH, -COOR₂, -NHR₃, -CONHR₄, ou/et -O-CO-R₄, un alkyle en C₄-C₂₀ substitué par OH et interrompu par un ou plusieurs atomes d'oxygène, un propyle avec comme substituants des OH et alcoxy en C₁-C₁₂ ou phénoxy, un cyclohexyle substitué par des groupe OH ou -OCOR₄, un allyle, un glycidyle ou l'un des groupes 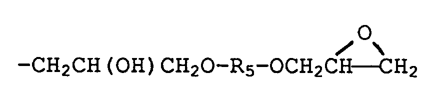 ou -CO-NH-R₇-NCO,
et si n vaut 2, R₁ sera l'un des groupes -CH₂CH(OH)CH₂-,
-CH₂CH(OH)CH₂O-R₅-OCH₂CH(OH)CH₂- ou
-CH₂CH(R₈)O-CO-CH=CH-CO-OCH(R₈)CH₂-,
R₂ désigne un alkyle en C₁-C₄, un glycidyle ou un allyle,
R₃ un alkyle en C₁-C₁₂,
R₄ un alcényle en C₂-C₆,
R₅ un alkylène en C₂-C₁₀, un phénylène ou un groupe 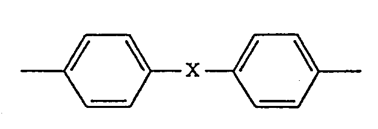 dans lequel X est -O-, -S-, -SO₂, -CH₂- ou -C(CH₃)₂-, ou bien R₅ est un groupe -CO-R₉-CO-,
R₇ un alkylène en C₂-C₁₀, un phénylène, un tolylène ou un groupe de formule 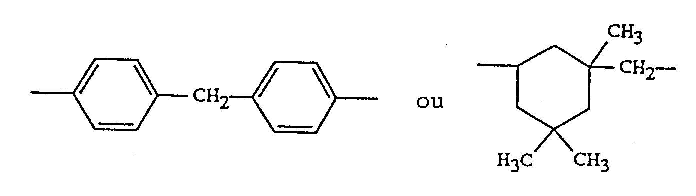 R₈ l'hydrogène ou le méthyle et
R₉ un alkylène en C₂-C₁₀, le groupe -CH=CH- ou un phénylène.

3. Procédé selon la revendication 1, caractérisé en ce que l'on incorpore un composé de formule I dans laquelle n vaut 1 ou 2,
A₁ et A₃ sont l'hydrogène, le méthyle ou le chlore,
A₂ et A₄ l'hydrogène ou le méthyle,
R₁, si n vaut 1, est l'hydrogène, un alkyle en C₁-C₄ avec comme substituants des OH, -COOH, -COOR₂, ou/et -O-CO-R₄, un alkyle en C₄-C₂₀ substitué par OH et interrompu par un ou plusieurs atomes d'oxygène, un propyle avec comme substituants des OH et alcoxy en C₄-C₁₂, un glycidyle, un allyle ou un groupe 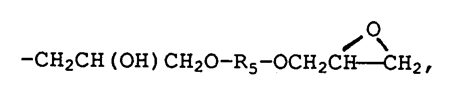 et n vaut 2, un des groupes-CH₂CH(OH)CH₂- ou -CH₂CH(OH)CH₂O-R₅-OCH₂CH(OH)CH₂-,
R₂ désigne un alkyle en C₁-C₄ ou un allyle,
R₄ un alcényle en C₂-C₄, et
R₅ un alkylène en C₄-C₈, ou 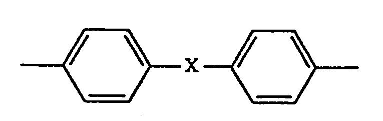 dans lequel X est -CH₂- ou -C(CH₃)₂-.

4. Procédé selon la revendication 1, caractérisé en ce que l'on copolymérise un composé de formule I dans laquelle n vaut 1 ou 2, R₁, si n vaut 1, est un alcényle en C₂-C₆, un alkyle en C₁-C₁₈ substitué par des -COOR₂, -NHR₃, -CONHR₄ ou -O-CO-R₄, un cyclohexyle avec comme substituant -O-CO-R₄ ou un groupe -CO-CH=CH-COOH, et si n vaut 2, est un groupe -CO-CH=CH-CO-, -CH₂CH(R₈)O-CO-CH=CH-CO-OCH(R₈)CH₂- ou
-CH₂CH(OH)CH₂O-CO-CH=CH-CO-OCH₂CH(OH)CH₂-, R₂ désigne un alcényle en C₃-C₅, R₃ un allyle, R₄ un alcényle en C₂-C₆, et R₈ l'hydrogène ou le méthyle, avec un ou plusieurs monomères à insaturation éthylénique.

5. Procédé selon la revendication 4, caractérisé en ce que les monomères employés sont l'acide acrylique, l'acide méthacrylique, des esters ou amides de l'acide acrylique ou méthacrylique, le styrène ou l'acrylonitrile.

6. Procédé selon la revendication 1, caractérisé en ce que l'on incorpore un composé de formule I dans laquelle n vaut 1 ou 2 et si n vaut 1, R₁ est un alkyle en C₁-C₁₈ substitué par des OH, -COOH, -COOR₂ ou -NHR₃, ou bien des -CH₂CH(OH)CH₂OH ou 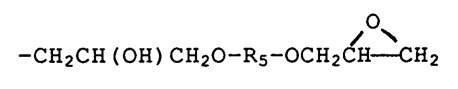 et si n vaut 2, un groupe -CH₂CH(OH)CH₂O-R₅-OCH₂CH(OH)CH₂-, R₂ désigne un alkyle en C₁-C₄, R₃ l'hydrogène, un alkyle en C₁-C₁₂, un allyle ou un cyclohexyle et R₅ un alkylène en C₂-C₁₀, un phénylène ou un groupe 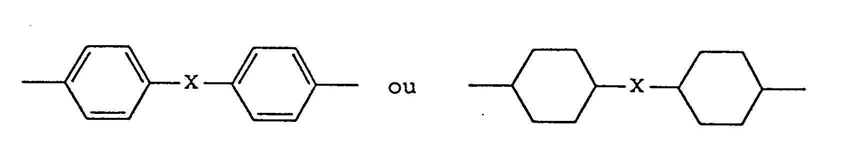 dans lequel X désigne -O-, -S-, -SO₂, -CH₂- ou -C(CH₃)₂-, par une copolycondensation ou une copolyaddition dans un polyester ou un polyétherester, un polyamide, un polyuréthanne, un polycarbonate, une résine époxyde, une résine phénolique, une résine de mélamine ou une résine alkyde.

7. Procédé selon la revendication 1, caractérisé en ce que l'on fait réagir un polymère qui contient des groupes hydroxyliques, carboxyliques, anhydrides, amino, époxydes ou isocyanates avec un composé de formule I contenant des groupes fonctionnels tels qu'ils peuvent réagir avec les groupes fonctionnels.

8. Procédé selon la revendication 1, caractérisé en ce que l'on greffe un composé de formule I à insaturation éthylénique tel que défini à la revendication 4 sur un polymère d'hydrocarbure.

9. Polymère modifié selon le procédé de la revendication 1, qui comprent de 0,05 à 50 % en poids d'un composé triazinique de formule I lié chimiquement au polymère.

10. Polymère modifié selon la revendication 9 qui contient au moins un additif physique pris parmi un stabilisant, un adjuvant de traitement, un pigment ou autres additifs.

11. Polymère modifié selon la revendication 9, qui contient une amine à empêchement stérique incorpée au polymère.

12. Utilisation d'un polymère modifié selon la revendication 9 comme liants de vernis.
